# EUROPÄISCHE PATENTANMELDUNG

(11) **EP 1 944 263 A1**
(43) Veröffentlichungstag der Anmeldung: **16.07.2008**
(21) Anmeldenummer: 07019677.9
(22) Anmeldetag: 09.10.2007
(51) Int. Cl.: B67C 3/22

(54) **Vorrichtung zum Behandeln von offenen Gefässen**

(30) Priorität: 18.12.2006 DE 202006019136 U
(71) Anmelder: KRONES AG, 93073 Neutraubling (DE)
(72) Erfinder: Söllner, Hannelore, 93183 Holzheim am Forst (DE)

(57) **Zusammenfassung**

Die Erfindung betrifft eine Vorrichtung zum Behandeln von offenen Gefäßen, insbesondere Flaschen, mit einer in die Gefäßöffnung einfahrbaren, an mindestens eine Gasleitung angeschlossenen Düse und einer dieser zugeordneten, den Gefäßkopf mit Abstand umgebenden Glocke zum Umlenken des aus der Gefäßöffnung austretenden Gases an den Gefäßkopf, wobei die als Leitfläche dienende Innenseite der Glocke im Wesentlichen kalottenförmig ist.

## Beschreibung

Die Erfindung betrifft eine Vorrichtung zum Behandeln von offenen Gefäßen, insbesondere Flaschen, gemäß dem Oberbegriff den Anspruchs 1.

Derartige Vorrichtungen werden insbesondere in Anlagen zum sterilen Befüllen von Kunststoff-Flaschen eingesetzt und dienen der Beaufschlagung des Flascheninneren mit Sterilluft, Heißluft, Sterilisationsgas oder dgl.. Mit Hilfe der Glocke wird das aus dem Ringspalt zwischen Düse und Flaschenöffnung austretende Gas umgelenkt und an der Außenseite des Flaschenkopfs entlang geleitet, um auch diesen mit zu behandeln.

Es ist bereits eine Vorrichtung der Eingangs genannten Art bekannt, bei der die Düse aus einem Rohr oder zwei konzentrischen Rohren besteht und mit einer zylindrischen oder sich zur offenen Seite hin konisch verjüngenden Glocke verbunden ist (WO 2006/053745). Die konische Glocke weist im Inneren zusätzlich ein konisches Leitblech auf, das den Gasstrom intensiv an den Flaschenkopf lenken soll.

Die Glocken der bekannten Vorrichtung erzeugen auf Grund ihrer Geometrie der als Leitfläche dienenden Innenseite und der dortigen Einbauten starke Turbulenzen in dem aus der Flaschenöffnung ausströmenden Gas, die die gewünschte Wirkung stark beeinträchtigen. Besonders störend sind diese energievernichtenden Turbulenzen bei einer Erwärmung des relativ dickwandigen oder doppelwandigen Kopfs von Kunststoff-Flaschen mittels Heißluft. Auch die Reinigung und Keimfreihaltung der bekannten Vorrichtung ist auf Grund der Ecken, Kanten und Einbauten problematisch.

Der Erfindung liegt die Aufgabe zugrunde, bei einer Vorrichtung der Eingangs genannten Art mit einfachen Mitteln eine besonders wirksame Anströmung der Außenseite des Gefäßkopfs zu realisieren.

Diese Aufgabe wird erfindungsgemäß durch die im Kennzeichen des Anspruchs 1 angegebenen Merkmale gelöst.

Die kalottenförmige Gestaltung der als Leitfläche dienenden Innenfläche der Glocke ermöglicht eine weitgehend turbulenzfreie Umlenkung und Strömung des austretenden Gases, wobei sich im Bereich der Gefäßöffnung ein gewisser Stau ergibt, so dass das Gas nicht sofort entspannt wird, sondern seine Energie behält.

Vorteilhafte Weiterbildungen der Erfindung sind in den Unteransprüchen angegeben.

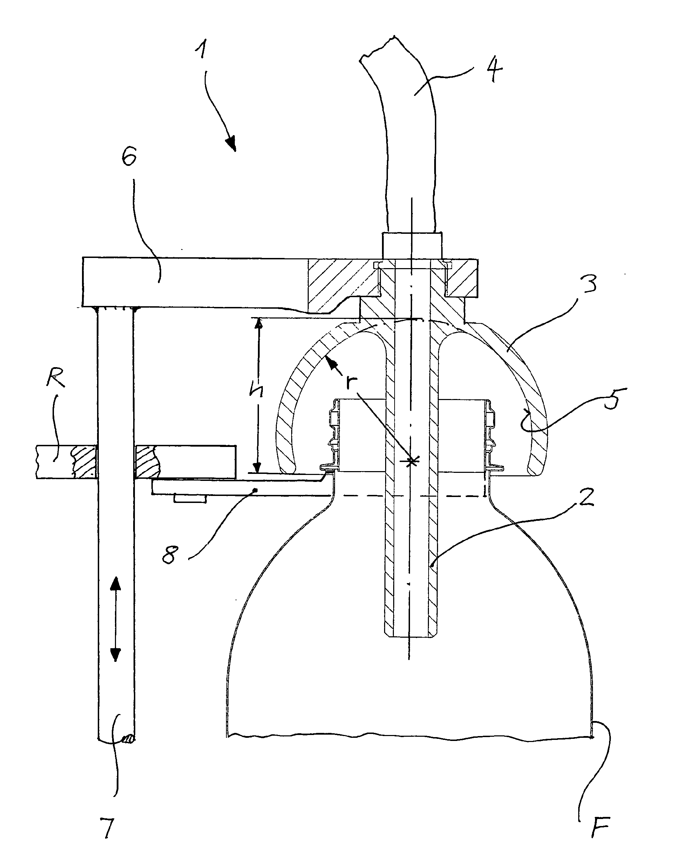

Im Nachstehenden wird ein Ausführungsbeispiel der Erfindung anhand der Zeichnung beschrieben. Diese zeigt einen senkrechten Schnitt durch eine Vorrichtung 1 zum Erwärmen von Kunststoff-Flaschen F.

Die Vorrichtung 1 weist einen horizontalen Träger 6 auf, an dessen einem Ende eine senkrecht stehende, rohrförmige Düse 2 befestigt ist. Diese ist über eine flexible Gasleitung 4 und ein nicht gezeigtes Steuerventil mit einer Quelle für sterile Heißluft verbunden. Das andere Ende des Trägers 6 ist an einer senkrechten, höhenbeweglichen Stange 7 befestigt, die durch einen nicht gezeigten Hubantrieb auf- und abbewegt werden kann.

Am oberen Ende der Düse 2 dicht unterhalb des Trägers 6 ist eine rotationssymmetrische Glocke 3 konzentrisch zur Düse 2 befestigt oder einstückig mit dieser ausgebildet. Die Innenseite 5 der Glocke 3 hat die Form einer Kalotte mit dem Radius r. Die Schnittebene der Kalotte verläuft horizontal und definiert die untere Öffnung der Glocke 3. Am oberen Endbereich geht die Innenseite 5 der Glocke 3 abgerundet in die zylindrische Außenseite der Düse 2 über. Auch die Außenseite der Glocke 3 ist überwiegend kalottenförmig.

Die vorbeschriebene Vorrichtung 1 ist zusammen mit einer Anzahl identischer Vorrichtungen auf dem Umfang eines Rotors R angeordnet, der um eine senkrechte Achse umläuft. An dem Rotor R ist für jede Vorrichtung 1 ein radial nach außen stehender Gefäßhalter 8 angeordnet, beispielsweise in Form einer gefederten Klammer, wie sie im deutschen Gebrauchsmuster 297 13 510 beschrieben ist. Durch den Gefäßhalter 8 wird eine Flasche F unterhalb ihres Tragrings ergriffen und vorübergehend konzentrisch zur zugehörigen Düse 2 fixiert. Während des Ein- und Ausführens einer Flasche F in einen Gefäßhalter 8 ist die Düse 2 mittels der Stange 7 entsprechend angehoben; nach dem Einsetzen einer zu behandelnden Flasche F in den Gefäßhalter 8 wird die Düse 2 zusammen mit ihrer Glocke 3 in die dargestellte untere End- und Arbeitsposition abgesenkt.

In der Arbeitsposition liegt die Öffnung der Glocke 3 geringfügig tiefer als der Tragring der Flasche F und es besteht ein vorgegebener Abstand von wenigen mm zwischen der Flaschenöffnung und der Außenseite des mit einem Gewinde versehenen Flaschenkopfs.

Wie die Zeichnung zeigt, ist die Höhe h der Kalotte geringfügig größer als ihr Radius r, so dass der untere, freie Rand der als Strömungs-Leitfläche dienenden Innenseite 5 leicht nach innen gewölbt ist. Hierdurch wird eine besonders gut Anströmung des Flaschenkopfs erzielt.

Mit der vorbeschriebenen Vorrichtung 1 kann eine Flasche F in kurzer Zeit durch Einblasen von Heißluft gleichmäßig erwärmt werden und zwar sowohl im relativ dünnen Wandbereich als auch im relativ dicken bzw. doppelwandigen Kopfbereich, da die erfindungsgemäße kalottenförmige Gestaltung der Glocke 3 für eine effektive Erwärmung des Flaschenkopfs von außen her sorgt.

Entsprechend vorteilhaft ist die Wirkung auch bei der Behandlung mit einem gasförmigen Sterilisationsmittel, z. B. einem Gemisch aus Luft und Wasserstoffperoxid, sowie bei anderen Düsenformen oder geringfügigen Abweichungen von der reinen Kalottenform, die zudem aufgrund ihrer "glatten" Gestalt die Reinigung begünstigt.

## Patentansprüche

1. Vorrichtung (1) zum Behandeln von offenen Gefäßen, insbesondere Flaschen (F), mit einer in die Gefäßöffnung einfahrbaren, an mindestens eine Gasleitung (4) angeschlossenen Düse (2) und einer dieser zugeordneten, den Gefäßkopf mit Abstand umgebenden Glocke (3) zum Umlenken des aus der Gefäßöffnung austretenden Gases an den Gefäßkopf, **dadurch gekennzeichnet, dass** die als Leitfläche dienende Innenseite der Glocke (3) im Wesentlichen kalottenförmig ist.

2. Vorrichtung nach Anspruch 1, **dadurch gekennzeichnet, dass** die kalottenförmige Innenseite (5) der Glocke (3) konzentrisch zu der rohrartigen Düse (2) angeordnet ist.

3. Vorrichtung nach Anspruch 2, **dadurch gekennzeichnet, dass** die kalottenförmige Innenseite (5) der Glocke (3) abgerundet in die Außenseite der rohrartigen Düse (2) übergeht.

4. Vorrichtung nach einem der Ansprüche 1 bis 3, **dadurch gekennzeichnet, dass** die Glocke (3) fest mit der Düse (2) verbunden ist.

5. Vorrichtung nach einem der Ansprüche 1 bis 4, **dadurch gekennzeichnet, dass** die Höhe der Kalotte größer ist als ihr Radius, so dass der die Öffnung bildende freie Rand der Innenseite (5) nach innen gewölbt ist.
